# EUROPEAN PATENT SPECIFICATION

(11) **EP 3 871 637 B1**
(45) Date of publication and mention of the grant of the patent: **16.04.2025**
(21) Application number: 19877410.1
(22) Date of filing: 21.10.2019
(51) Int. Cl.: A61F 2/44, A61L 27/04, A61L 27/10, A61L 27/14, A61F 2/30, A61L 27/50

(54) **IMPLANT MATERIAL AND METHOD FOR PRODUCING SAID IMPLANT MATERIAL**
IMPLANTATMATERIAL UND VERFAHREN ZUR HERSTELLUNG DES IMPLANTATMATERIALS
MATÉRIAU D'IMPLANT ET PROCÉDÉ DE FABRICATION DUDIT MATÉRIAU D'IMPLANT

(30) Priority: 23.10.2018 JP 2018199592
(43) Date of publication of application: 01.09.2021
(73) Proprietor: Osaka University, Suita-shi, Osaka 565-0871 (JP); Teijin Nakashima Medical Co., Ltd., Okayama-shi Okayama 709-0625 (JP)
(72) Inventor: NAKANO, Takayoshi, Suita-shi, Osaka 565-0871 (JP); ISHIMOTO, Takuya, Suita-shi, Osaka 565-0871 (JP); TAKAHASHI, Hiroyuki, Okayama-city Okayama 709-0625 (JP); INOUE, Takayuki, Okayama-city Okayama 709-0625 (JP); SUMASU, Yasuki, Okayama-city Okayama 709-0625 (JP); UETUKI, Keita, Okayama-city Okayama 709-0625 (JP); KIMURA, Hiroomi, Okayama-city Okayama 709-0625 (JP)
(74) Representative: Studio Torta S.p.A.
(86) International application number: PCT/JP2019/041360
(87) International publication number: WO 2020/085321

(56) References cited:
- WO-A1-2017/106780
- WO-A1-2018/165405
- WO-A1-2019/140240
- CN-A- 103 445 883
- JP-A- 2015 529 150
- JP-U- 3 181 826
- US-A1- 2017 360 563

## Description

### Technical Field

The present invention relates to an implant material and a method for manufacturing the implant material, and more particularly to an implant material having grooves and unidirectional holes and a method for manufacturing the implant material.

### Background Art

Conventionally, various bioimplant materials have been proposed as alternative materials for bone. For example, high-strength materials such as stainless alloys, titanium-based metals such as titanium and titanium alloys, and bioactive materials such as apatite sintered bodies, bioactive glass, and bioactive crystallized glass are known, see for example patent document WO2017/106780A1.

High-strength materials such as stainless alloys and titanium-based metals have the characteristic of having high mechanical strength, but they do not directly adhere to bone as they are. Further, bioactive materials such as apatite sintered body, bioactive glass, and bioactive crystallized glass bind to bone in a short period of time, but have a problem that they are insufficient in strength and the applicable place is limited. In order to solve these problems, an implant material in which a film made of a bioactive material is formed on the surface of a high-strength material by plasma spraying or baking has been proposed (Japanese Unexamined Patent Publication No. H8-357040). In this way, bone implants caused by bone diseases, bone defects, etc. are frequently used, and it is expected that demand will increase with the progress of an aging society.

Further, taking the human spine as an example of bones, for example, the spine plays an important role in supporting the trunk and protecting the nerves (spinal cord) that transmit the sensations and movements of the internal organs and limbs from the brain. However, if the vertebrae that make up the spine or the intervertebral discs that support load and move between the vertebrae are deformed due to a disease, there are possibilities that the deformed vertebrae or intervertebral discs may press the spinal cord. In this case, the compression of the spinal cord causes symptoms such as numbness and pain in the limbs.

Surgery to insert a spacer (generally called a cage) into a portion of the intervertebral disc between the vertebrae is performed for the purpose of relieving the compression of the spinal cord. By inserting a cage between the vertebrae and mechanically immobilizing it, it is intended to reproduce the appropriate spacing and position between the vertebrae and to relieve the compression of the spinal cord.

In general, this cage has a role of supporting the load caused by the weight, and since it is made of metal or resin having high mechanical strength, it does not directly adhere to the bone of the living body. Therefore, a method is adopted in which a through hole is provided in the cage, and a bone is guided into the hole to be fixed by an anchoring effect. In addition, screws and rods may be used to connect the vertebrae to prevent them from moving.

Although the cage is highly useful in this way, the fixation between the vertebrae and the cage is not sufficient, and the cage moves and falls out from between the vertebral bodies after surgery, or the cage moves between the vertebral bodies, damaging the vertebrae and surrounding tissues. As a result, the spinal cord is compressed again, and the cage interferes with other parts to induce numbness and pain, which requires surgery again.

### Prior art literature

### Patent literature

Patent literature 1 : JP-A1-H8-357040

### Disclosure of the Invention

### Problems to be resolved by the invention

However, many of the above-mentioned conventional implant materials pay attention to the mechanical properties of the material itself, and there are few materials that are conscious of the fine structure of the bone itself, which plays a leading role in the anchoring effect. The porous body of the prior art is intended to vaguely expect bone invasion into the pores (holes, or opening), and is not intended to form an excellent tissue that prevents deterioration of bone mass and bone quality (here, which represents bone strength). Therefore, an implant material that realizes high-quality bone invasion in consideration of bone mass and bone quality has been desired.

Further, in addition to the above method, a method has been adopted in which bone is separately collected from the ilium (the ilium is a part of the pelvic) so that the bone can quickly enter the through hole of the implant material such as the cage and be firmly fixed, and then the collected bone is transplanted into the through hole of the cage to promote the induction of the bone into the through hole. Since additional skin incisions will be added in addition to the back, which is the target of surgery, the burden on the patient will increase and there will be concerns about pain. In addition, even when transplanted bone is used, there is possibility that the implant material such as a cage may not be sufficiently fixed. Emphasis was placed on attracting bone into the hole of the implant material such as a cage, and the quality of the bone mass and the quality of the bone that invaded the hole were not sufficiently examined as described above. In particular, it is known that the bone that has just invaded the hole has a high bone density but a weak mechanical strength, and it is considered that the initial fixation of the implant material such as a cage is low.

In addition, it is required that implant materials such as cages not only guide bone near the surface like artificial joints, but also deeply invade and fill bone in the thickness direction (the thickness direction corresponds to, for example, the intervertebral space direction). In order to improve the fixation of implant materials such as cages, it is necessary to consider the bone quality in the deep part of the cage hole. It is well known that bone quality is related to mechanical stimulation in vivo due to weight, etc., but since the rigidity of cages made by metal or PEEK is larger than that of bone, it is difficult for the load to be transmitted to the bone. Especially it becomes difficult to set the external environment such as mechanical stimulation in the deep part of the hole.

Therefore, an object of the present invention is to provide an implant material having improved fixation with a living body.

### Means of solving the problems

In order to achieve the above object, the inventors focused on the structure of the original hard tissue existing in the living body, and as a result of diligent research on its application to the implant material. As a result, the inventors discovered the implant material of the present invention.

An implant material according to the present invention, is characterized by comprising a hole in at least one direction, and a member constituting the hole comprising grooves, the grooves being provided on the surface of hole in the depth direction of the hole.

Further, in a preferred embodiment of the implant material according to the present invention, it is characterized in that the member constituting the hole is composed of pillar and/or plate.

Further, in a preferred embodiment of the implant material according to the present invention, it is characterized in that the grooves are provided in the pillars and/or the plates.

Further, in a preferred embodiment of the implant material according to the present invention, it is characterized in that the multiple holes are composed of pillars and / or plates.

Further, in a preferred embodiment of the implant material according to the present invention, it is characterized in that the grooves are alternately arranged on the front and back surfaces of the pillar and / or the plate.

Further, in a preferred embodiment of the implant material according to the present invention, it is characterized in that the pillar and / or the plate are composed of a simple unit and / or a block.

Further, in a preferred embodiment of the implant material according to the present invention, it is characterized in that the pillar and / or plate are designed to bend.

Further, in a preferred embodiment of the implant material according to the present invention, it is characterized in that the hole is designed not to penetrate the implant material or is designed to penetrate the implant material.

Further, in a preferred embodiment of the implant material according to the present invention, it is characterized in that the implant material is at least one selected from a polymer material, a ceramic material, a metal material, an amorphous material, or a mixed material thereof.

Further, in a preferred embodiment of the implant material according to the present invention, it is characterized in that a width of the groove is 0.25 to 500 µm.

Further, in a preferred embodiment of the implant material according to the present invention, it is characterized in that the holes are in communication with each other when there are a plurality of holes.

Further, in a preferred embodiment of the implant material according to the present invention, it is characterized in that pillars and / or plates are designed so that the pillars and / or plates are bend by having a stretchable structure by themselves, or by varying the thickness, width, or height of the pillars or plates.

Further, in a preferred embodiment of the implant material according to the present invention, it is characterized in that the implant material is designed to bend at least a part of the contact surface where the implant material and a living body come into contact with each other.

Further, in a preferred embodiment of the implant material according to the present invention, it is characterized in that the hole is formed of a truss structure body and the inscribed circle of the hole is 500 µm to 2000 µm.

Further, in a preferred embodiment of the implant material according to the present invention, it is characterized in that the implant material has a cage-like structure and has a second hole on a side surface of the cage-like structure.

Further, a method of manufacturing the implant material according to the present invention, is characterized by being a method for manufacturing the implant material of the present invention, and is characterized by being produced by a 3D modeling method.

### Effect of Invention

According to the implant material of the present invention, it has advantageous effects that early bone entry and early fixation are possible, and an adverse effect on surrounding bone is reduced. Further, the implant material according to the present invention has advantageous effects that strong immobilization between the living body and the implant material can be realized at an early stage without requiring setting of an external environment such as a mechanical stimulus or a magnetic field.

Further, according to the method of manufacturing an implant material of the present invention, it is possible to provide an implant material capable of early bone entry and early fixation and reducing adverse effects on surrounding bone.

### [Brief explanation of drawings]

[Fig. 1]

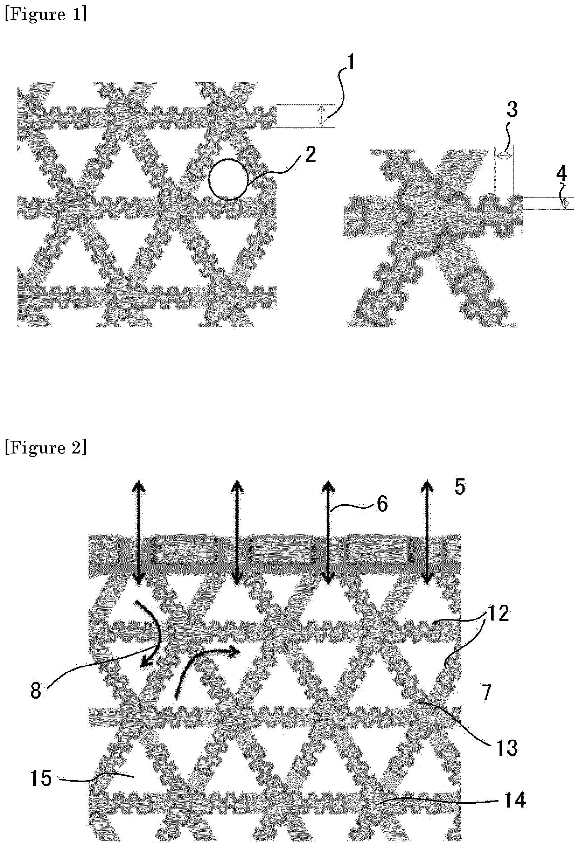

   FIG. 1 shows a cutaway view of an example of a member constituting the hole. It can be seen that the members constituting the holes have grooves. Although it is a cross section, it also includes a shape (a truss structure in addition to the tetra-shaped porous body inside) that is on the other side of the cross section in a split state.
[FIG. 2]
   FIG. 2 shows an example of the relationship between the members constituting the hole and the outer periphery of the implant material.
[FIG. 3]

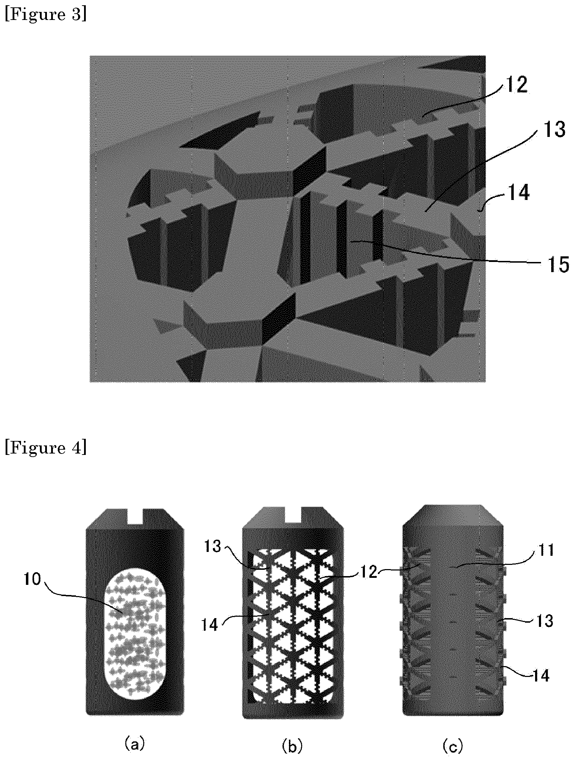

   FIG. 3 shows a perspective view of an example of the truss structure and the groove.
[FIG. 4]
   FIG. 4 (a) shows a conventional implant material (Evaluation sample C). FIG. 4 (b) shows an implant material according to an embodiment of the present invention in which a unidirectional hole penetrates (Evaluation sample D). FIG. 4 (c) shows a view of the implant material of FIG. 4 (b) as viewed from an angle rotated by 90 degrees, and a hole is designed on the side surface of the cage. (Evaluation sample E).
[FIG. 5]

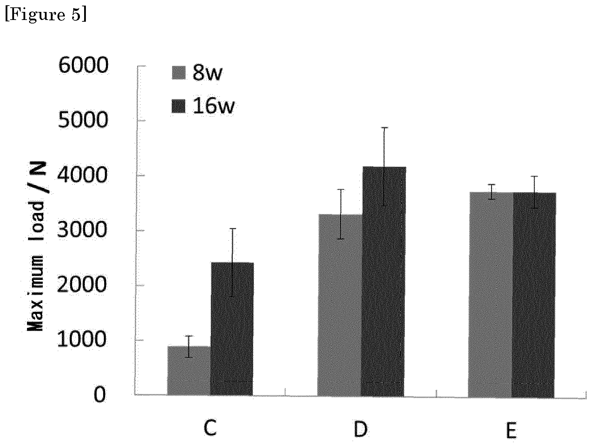

   FIG. 5 shows the result of the pull-out test with the measured maximum load as the pull-out strength.
[FIG. 6]

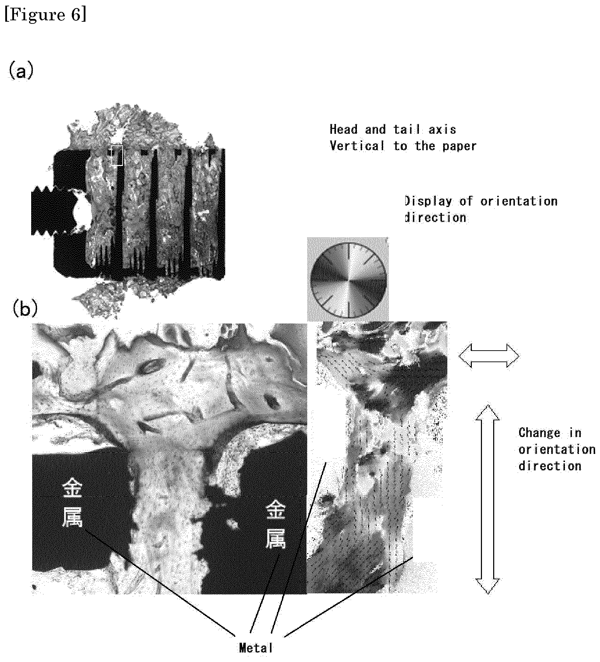

   FIG. 6 shows the results of microscopic observation of evaluation material E. FIG. 6 (b) shows an enlarged view of a portion surrounded by a rectangle on the left side of the center of FIG. 6 (a).
[FIG. 7]

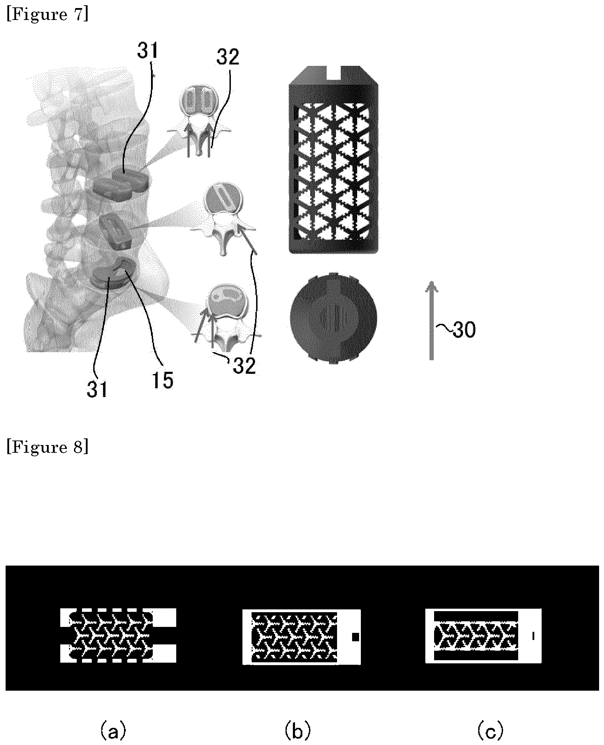

   FIG. 7 shows an example of using the implant material in one embodiment of the present invention.
[FIG. 8]
   FIG. 8 shows a schematic view of the implant material according to one embodiment of the present invention when a cylindrical cage is used for the vertebral body. FIG. 8 (a) shows the central portion of the cylindrical cross section, FIG. 8 (b) shows the intermediate portion located between the central portion and the upper portion of the cylindrical cross section, and FIG. 8 (c) shows the upper portion of the cylindrical cross section.
[FIG. 9]

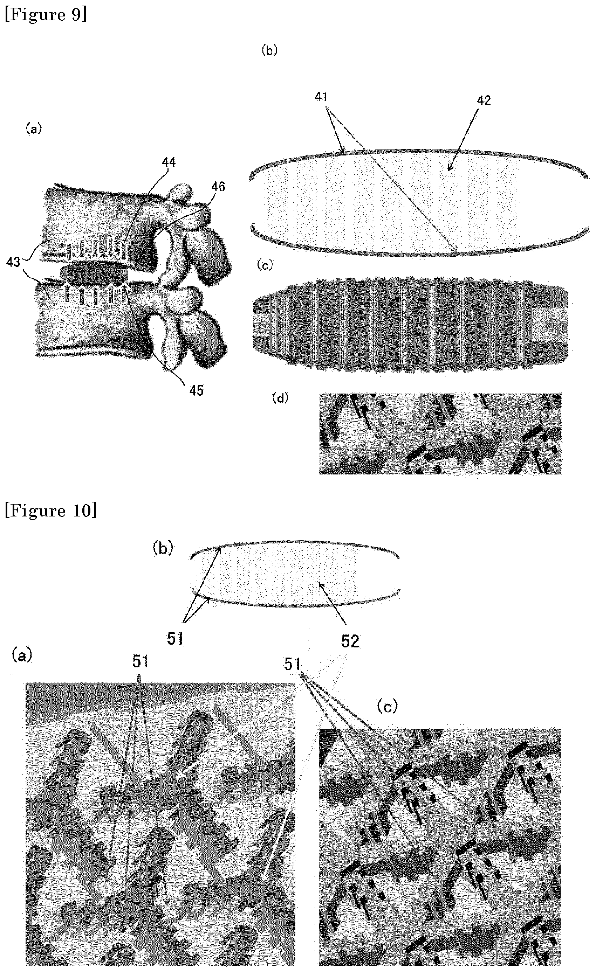

   FIG. 9 shows a schematic view of the implant material according to one embodiment of the present invention when a box-shaped cage used between intervertebral is used for the vertebral body. FIG. 9 (a) is a view seen from the lateral direction (direction perpendicular to the cranio-caudal direction) when an example of the implant material of the present invention is incorporated into the spine. FIG. 9 (b) is a schematic view of an example of the implant material of the present invention as viewed from the lateral direction (direction perpendicular to the cranio-caudal direction). 41 in FIG. 9 (b) shows the ceiling structure of the box-shaped cage that forms a contact surface with the vertebral body and the end plate in the vertical direction. FIG. 9 (c) is a cross-sectional view of an example of the implant material of the present invention as viewed from the lateral direction (direction perpendicular to the cranio-caudal direction). FIG. 9 (d) is a perspective view (viewed from diagonally above) of an example of the implant material of the present invention.
[FIG. 10]
   FIG. 10 shows a schematic view of a box-shaped cage in the implant material according to one embodiment of the present invention. FIG. 10 (a) is a cross-sectional view of an example of the implant material of the present invention. FIG. 10 (b) is a schematic view of an example of the implant material of the present invention as viewed from the lateral direction (direction perpendicular to the cranio-caudal direction). 51 in FIG. 10 (b) shows the ceiling structure of the box-shaped cage that forms a contact surface with the vertebral body and the end plate in the vertical direction. FIG. 10 (c) is a perspective view of an example of the implant material of the present invention.
[FIG. 11]

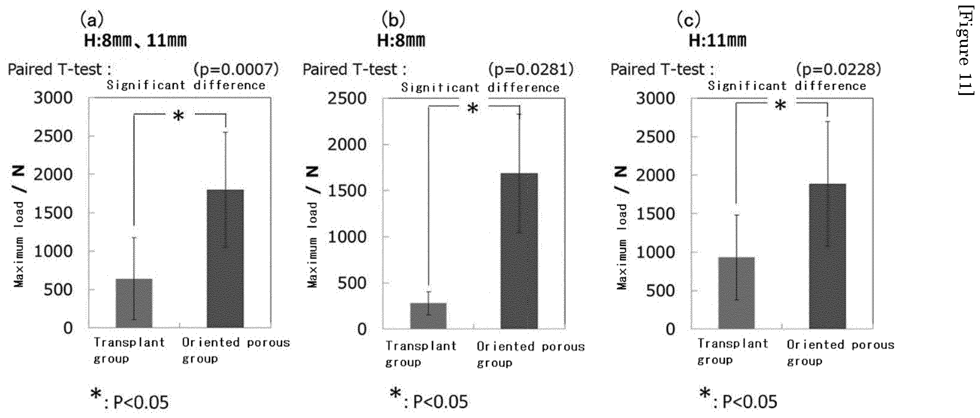

   FIG. 11 shows the result of having carried out the comparative extrusion test of the transplanted bone group of the box-shaped cage equivalent to the evaluation sample C of FIG. 4 (a) and the bone orientation induction group of the box-shaped cage equivalent to the evaluation sample D of FIG. 4 (b) for the two types of box-shaped cage heights of 8 mm and 11 mm. FIG. 11 (a) shows the result when both the box-shaped cage heights of 8 mm and 11 mm are combined, FIG. 11 (b) shows the result when a height of the box-shaped cage is 8 mm, and FIG. 11 (c) shows the results when the height of the box-shaped cage is 11 mm are shown, respectively.

### Mode for Carrying Out the Invention

An implant material according to the present invention, is characterized by comprising a hole in at least one direction, and a member constituting the hole comprising grooves being provided on the surface of the hole in the depth direction. The groove makes it possible to extend and arrange osteoblasts in the depth direction (the thickness direction of the cage when a cage is used as described later) of the holes (pores or opening) of the implant material when the osteoblasts first invade the inside of the implant material (porous body). In the present invention, by having the groove in addition to the pore, the arranged osteoblasts produce a bone matrix oriented in parallel with the extension / arrangement direction, so that it has the effect of promoting bone matrix orientation from the beginning that the osteoblasts are implanted without mechanical stimulation (bone regeneration). Further, in the present invention, by setting a groove other than the hole, it is possible to obtain strong fixation at an early stage between the bone and the implant material without setting an external environment such as a mechanical stimulus or a magnetic field.

In the present invention, the groove is not particularly limited, but for example, a groove can be set on the surface of a pillar (or a column) or a plate described later. The width of the groove is also not particularly limited as long as it is set for the member constituting the hole, but the width of the groove can be preferably 0.25 µm to 500 µm, and more preferably 0.5 to 200 µm. Further, the grooves can be provided at equal intervals in the thickness direction described above.

Further, in a preferred embodiment of the implant material of the present invention, the member constituting the hole is characterized by being composed of a pillar and / or a plate. As described above, the structure of the pillar and / or the plate makes it possible to easily form a porous body inside the implant material. The present invention can easily provide a porous body that induces bone invasion and bone orientation in a hole inside an implant material such as a cage in order to obtain strong fixation between vertebrae at an early stage without requiring setting of an external environment. In the porous body, pillars or / and plates can be arranged in a pattern at regular intervals in the thickness direction of the cage (in the case of forming a hole, the direction of the major axis of the hole; in the case of a vertebra, the direction of the cranio-caudal axis).

In this way, the rigidity of the pillar can be adjusted by changing the thickness, width, height, etc. of the pillar or the plate. That is, in a preferred embodiment of the implant material of the present invention, from the viewpoint of maintaining good bone quality for a long period of time by applying continuous mechanical stimulation after forming oriented bone in the porous body, it is characterized in that the pillar and / or the plate are designed to bend (flex) by having a stretchable structure itself or by changing the thickness, width, or height of the pillar or the plate. Here, the stretchable structure is not particularly limited as long as the stretchable structure expands and contracts. For example, in the case of insertion into a vertebral body, the stretchable structure can mean that it contracts when a load is received from the upper and lower vertebral bodies and returns to its original state when a load is not applied.

Further, in a preferred embodiment of the implant material of the present invention, from the viewpoint that the living body and the implant material are more compatible with each other, it is characterized that the implant material is designed to bend (or flex) at least a part of the contact surface where the implant material and the living body come into contact with each other. By designing to bend in this way, for example, when the implant material of the present invention is used as a facet cage for the vertebra, as will be clear in the examples described later, by bending, it can be expected that the contact surface with the vertebra, that is, front and back surfaces of the facet cage will fit into the shape of the bone. By fitting into the shape of the bone, the front and back surfaces of the cage are in close contact with the vertebrae, making it easier to guide the bone into the porous body. This is also clear from the fact that the bone is vigorously invaded into the porous body as a result of implanting the intervertebral cage in the intervertebral space of the sheep in the same way and observing the punching strength and tissue as in the examples described later.

Further, the thickness of the pillar or plate is not particularly limited. When the material of the implant material is, for example, a metal material, the thickness of the pillar or plate can be preferably 0.1 to 2 mm, more preferably 0.5 to 1 mm. although it depends on the material used, within this thickness range, the pillar or plate can easily flex in the thickness direction of the implant material such as the cage due to the load acting between the bones such as the vertebrae, thereby transmitting the load to the bone in the porous body. After the bone is oriented inside the porous body in this way, by receiving the main stress load (that is, mechanical stimulation) from the upper and lower vertebral bodies, the bone inside the cage can maintain and promote bone orientation, and it is possible to further suppress deterioration during long-term implantation. This makes it possible to maintain good bone quality in the porous body of the cage for a long period of time immediately after surgery, and as a result, to achieve stable fixation of the vertebrae and cage for a long period of time from the initial stage.

Further, in a preferred embodiment of the implant material according to the present invention, it is characterized in that the pillar and / or the plate are composed of a simple unit and / or a block. For example, the pillar and / or plate can be a block or a single unit connected in a radial, cross-shaped, staggered shape. The plate may be rotatable with the pillar as the center of rotation, and the size of the hole can be freely designed by rotating the plate and fixing the position at regular intervals.

Further, in a preferred embodiment of the implant material according to the present invention, from the viewpoint that bone orientation attains in the entire region where the bone penetrates to obtain strong fixation, it is characterized in that the multiple holes are composed of pillars and / or plates.

Further, in a preferred embodiment of the implant material according to the present invention, from the viewpoint that the pillar or / and plate defines the hole direction and orients the bone in that direction, it is characterized in that the grooves are provided in the pillars and/or the plates. Further, from the viewpoint of promoting bone matrix orientation from the initial stage of implantation (bone regeneration) without mechanical stimulation, the groove is provided along the depth direction (major axis direction) of the hole.

Further, in a preferred embodiment of the implant material according to the present invention, from the view point that strong fixation is obtained by reducing the plate thickness to minimize the volume of the artificial material such as metal occupying the porous body and maximizing the space where bone can penetrate, it is characterized in that the grooves are alternately arranged on the front and back surfaces of the pillar and / or the plate. For example, as an example of the reason why the grooves are provided alternately on the front and back of the plate, although the metal part is thinned to widen the area inside the cage that can be filled with bone and the plate thickness is reduced to increase the bone mass (to give flexibility), this is because there is possibility that the depth of the groove may not be secured unless the grooves are arranged alternately.

Further, in a preferred embodiment of the implant material according to the present invention, it is characterized in that the pillar and / or plate are designed to bend. Further, in a preferred embodiment, the pattern may be arranged by the pillars and the boards. This is because the pattern arrangement makes it possible to easily design according to the size of the holes. That is, it is possible to make the size of the hole composed of the pillar and the plate, that is, the size of the inscribed circle constant. For example, in the examples described later, although the optimum value of the inscribed circle of the hole is set to 500 µm or more in the sheep implantation test, but in order to easily define this optimum value, a space of a certain size can be created as a pattern arrangement. Since it is sufficient to provide a space larger than that, it may be random (there is no problem that the size of the inscribed circle is different) and is not particularly limited.

In a preferred embodiment of the implant material according to the present invention, from the viewpoint of maximizing the space in which the bone penetrates and obtaining a strong fixing force, it is characterized in that the hole is formed of a truss structure body and the inscribed circle of the hole is 500 µm to 2000 µm.

Further, as described above, the rigidity of the pillar can be adjusted by changing the thickness, width, height, etc. of the pillar or the plate. Further, assuming that the shaft connecting between the pillars, the plates (boards), the plates and the plillars, etc. is a beam (girder) (when a cage is used, the portion corresponding to the ceiling (upper and lower sides) of the cage is included), the beam may be a flexible structure or a stretchable structure. It is similar to the expansion and contraction of the plate as described above. For example, by expanding and contracting the beams arranged in a honeycomb shape or the like, it is possible to adjust so that the contact surface of the box-shaped cage is in perfect contact with the shape of the end plate of the vertebral body. The thickness of the beam can be 0.1 mm to 1.5 mm, preferably 0.3 mm to 1.0 mm, and for example, the beam can be about 0.5 mm, from the viewpoint of the flexible structure.

Further, in a preferred embodiment of the implant material of the present invention, it is characterized in that the hole is designed not to penetrate the implant material or is designed to penetrate the implant material. In the present invention, when the osteoblasts first invade the implant material (porous body), the groove makes it possible to extend and arrange osteoblasts in the depth direction of the holes of the implant material for osteoblast (when a cage is used as described later, in the thickness direction of the cage), so that the hole may be a through hole or a non-penetrating hole. In the case the hole is designed to penetrate the implant material, the bone tissue is continuous between the adjacent vertebrae through a penetrating hole, so that the fixation strength between the vertebrae and the cage can be further secured.

The material of the implant material is not particularly limited. Further, in a preferred embodiment of the implant material of the present invention, as the implant material mention may be made of at least one selected from polytetrafluoroethylene ((Teflon (registered trademark)), polymer material, ceramic material, metal material, amorphous material, or a mixture thereof. As the metal material, mention may be made of pure metals, alloys, intermetal compounds and the like. Further, the amorphous material can include a partially crystallized portion. This is because there is a material called an amorphous material even if it contains a crystallized portion. As the amorphous material, for example mention may be made of bioglass and the like.

For example, as the material of the implant material, a hard tissue substitute material or the like can be mentioned. As the hard tissue substitute material, mention may be made of inorganic materials such as ceramics typified by apatite, alumina and zirconia, and metal materials such as stainless steel, Co-Cr alloy, titanium, alloy and tantalum. Ceramics can be further divided into bioactive ceramics, bioinactive ceramics and the like. As bioceramics, mention may be made of calcium phosphate-based ceramics, silica-based glass, and crystallized glass. Hydroxyapatite and tricalcium phosphate are well known as calcium phosphate-based ceramics, and these are used for artificial tooth roots, skin terminals, metal coating materials, and the like. These various materials can be used as the implant material.

Further, in a preferred embodiment of the implant material according to the present invention, from the viewpoint of the width at which osteoblasts can detect the orientation direction of collagen and apatite, a width of the groove can be preferably 0.25 to 500 µm, more preferably 0.5 to 200 µm.

Further, in a preferred embodiment of the implant material according to the present invention, it is characterized in that the holes are in communication with each other when there are a plurality of holes. This is because, for example, continuous inflow of bone marrow fluid is possible between the holes, and it is possible to realize bone invasion into the deep part of the porous body.

Further, a method of manufacturing the implant material according to the present invention, is characterized by being a method for manufacturing the implant material of the present invention, and is characterized by being produced by a 3D modeling method (AM, Additive Manufacturing). As for the 3D modeling method, a conventional method can be used and is not particularly limited. The processing method of the implant material and the like are widely known in the art, and the implant material can be produced by applying to the implant material of the present invention by a conventional method.

Further, the pillars or plates constituting the above-mentioned porous body can be connected to the truss structure body. The truss structure body may be a structure body connected to a triangular, square or polygonal shape on the upper and lower surfaces of the cage continuous with the structure body on the outer periphery of the cage. With this truss structure, the outer periphery of the cage and the pillars or plates constituting the porous body can be integrated. The size of the inscribed circle with respect to the void formed inside the triangular, square or polygonal shape of the truss structure body is also not particularly limited. For example, from the viewpoint of the size of bone lineage cells (osteoblast + osteoclast + osteosite), the size of the inscribed circle is preferably 500 µm or more. The pillars or plates constituting the porous body can be arranged along the truss of the truss structure body. In this case, the inscribed circle of the void formed by the arranged pllars and / or plates can also be equivalent to the inscribed circle of the truss porous body. The size of this void can be the optimum size for bone invasion into the porous body.

The truss structure itself may also be provided so that the same grooves as those provided in the pillars or plates constituting the porous body are continuous. The groove of this truss structure body makes it possible to promote bone orientation immediately after bone invasion, to develop high bone quality from the initial stage of bone invasion, and to realize cage fixation with vertebrae.

The pillars and / or pillars arranged to form the porous body may not be connected each other. In this case, spaces at regular intervals can be provided between the pillars, allowing continuous inflow of bone marrow fluid and realizing bone invasion into the deep part of the porous body. Further, a hole may be provided so as to communicate the outside of the outer periphery of the cage with the inside of the porous body. As a result, continuous inflow of bone marrow fluid is possible, and bone invasion into the deep part of the porous body can be realized.

Further, in a preferred embodiment of the implant material according to the present invention, from the viewpoint that the side holes are effective for guiding the bone into the porous body. it is characterized in that the implant material has a cage-like structure and has a second hole on a side surface of the cage-like structure. This is because it turned out to be effective for the hole on a side surface to guide the bone into the porous body from the data of the cylindrical cage (or box-shaped cage) embedded in the vertebrae regarding the hole on a side surface of the facet cage, as will be apparent in the examples described later.

### Example

Here, an embodiment of the present invention will be described, but the present invention is not construed as being limited to the following examples. Further, needless to say, it can be appropriately changed without departing from the scope of the present invention.

### Example 1

Bone tissue is composed of undifferentiated mesenchymal cells, osteogenic cells, osteoblasts, bone cells (osteocytes) and osteoclasts and the like. In the process of newborn bone formation, osteoblasts secrete type I collagen and the like, and additively produce apatite to collagen fibers to promote calcification. As calcification progresses, it becomes bone cells and is embedded in the bone matrix to complete newborn bone formation.

In such a bone formation process, it is known that the traveling direction of type I collagen (Col) and the crystal orientation of hexagonal apatite crystals (BAp) are almost the same (here, the orientation means that the orientation of the apatite crystals is not random but they are aligned in the same direction). This Col and BAp complex determines the bone matrix, that is, the strength and flexibility of the bone. Hexagonal crystals have a = b ≠ c as the crystal axis and show remarkable mechanical anisotropy along the a and c axes. Therefore, it can be seen that the mechanical properties of bone are closely related to the orientation of the bone matrix due to the orientation of the apatite crystals produced from the osteoblasts. In other words, it can be seen that osteoblasts and type I collagen and apatite secreted from them are closely related to their orientation. Therefore, in the present invention, in order to orient the bone matrix by extending and arranging the osteoblasts in the cage thickness direction of the osteoblasts, a groove structure extending in the orientation direction with respect to the porous body intended for bone invasion can be adopted.

In an example of the implant material prototyped this time, the grooves of the porous body are provided at equal intervals on the surface of the plate or / and the pillar constituting the porous body as shown in FIG. FIG. 1 shows a cross-sectional view of an example of a member constituting the hole. It can be seen that the members constituting the holes have grooves. In FIG. 1, it is shown that 1 is a plate thickness, 2 is an inscribed circle between plates, 3 is a groove width, and 4 is a groove depth, respectively. In this figure, the shape corresponding to the radial shape of 120 degrees indicates a tetra-shaped internal structure in which three plates are connected. The tetra-shaped structure is a tetra-shaped structure consisting of three plates, but may be cross-shaped. A cross is a structure in which two plates are crossed at 90 degrees. Further, the pillar can mean a central portion of a tetra-shaped structure, or a cylinder or a prism that stands perpendicular to the paper surface. The shape illustrated this time is only one example, and the arrangement of plates and pillars can be considered infinitely. FIG. 2 shows an example of the relationship between the members constituting the hole and the outer periphery of the implant material. FIG. 3 shows a perspective view of an example of the truss structure body and the groove. In FIG. 2, it is shown that 5 is the surrounding bone, 6 is the hole (second hole) communicating the surrounding bone and the inside of the implant material, 7 is the inside of the implant material (inside the porous body), and 8 is the hole communicating between the holes inside the implant material (third hole), 12 is a groove, 13 is a plate, 14 is a pillar, and 15 is a hole (first hole). In FIG. 3, it is shown that 12 is a groove, 13 is a plate, 14 is a pillar, and 15 is a hole (first hole), respectively. The hole 6 that communicates between the surrounding bone and the inside of the implant material (second hole) 6 and the hole 8 that communicates between the holes inside the implant material (third hole ) 8, for example, can also have a role of promoting continuous circulation of bone marrow fluid.

As shown in FIG. 2, as an example, the cage porous body can be formed into a tetra-shaped structure body by connecting the three radially spreading plates in which grooves are arranged at the center. The adjacent tetra-shaped structure bodies are not directly connected to each other, and a gap can be provided between the plates that spread radially to create a communicating porous body (8 in FIG. 2). When each tetra-shaped structure body is arranged with a gap in this way, nothing is restricted in the space and each tetra-shaped structure body falls off. Therefore, the tetra-shaped structure body and the structure body on the outer periphery of the cage can be connected and integrated by the plate-shaped structure bodies on the front and back surfaces of the cage. Further, for example, a tetra-shaped structure may be connected as a honeycomb structure, and a hole of a horizontal skewer (that is, a hole of a horizontal skewer perpendicular to the hole direction of the honeycomb, for example, 5 and 6 etc. in FIG. 2) may be formed in the honeycomb.

The finite width plates or / and pillars that make up the porous body are arranged in blocks that are connected in a radial or cross shape, or alone. Osteoblasts invade along the groove provided in this plate or / and pillar, and have a role of promoting bone orientation from the initial stage of bone invasion. The arrangement of the plates and / or pillars may be either staggered or evenly spaced. It is desirable that this plate or / and pillar bend or flex under load in a biomechanical environment. This deflection can provide mechanical stimulation in the direction of principal stress to the bone that has invaded the porous body along the plate or / and pillar, and can contribute to continuous bone orientation. Therefore, it is desirable that the thickness or thickness of the plate or / and the pillar be, for example, 1 mm or less so that the plate or the pillar can be bent or flexed by a load.

The outer peripheral part of the cage that forms the outer shape shares the load support in the in vivo mechanical environment, and at the same time, the porous body and the bone invading the porous body are integrated and the cage and the intervertebral space are fixed. That is, the outer peripheral portion of the cage and the porous body can be structurally integrated. As a method of connecting the space-arranged plate or / and the pillar and the outer circumference of the cage to form the porous body, a truss structure that connects the surface of the cage (that is, the upper and lower surfaces of the cage where the cage inserted between the intervertebral space contacts the vertebrae) and the outer circumference of the cage is arranged. This truss structure body is arranged so as to be connected to a plate or / and a pillar constituting the porous body. The truss shape may be any triangle, quadrangle, or polygon as long as it can be connected to a plate or a pillar constituting the porous body. The truss structure body on the surface of the cage is provided with a groove so as to be continuous with the groove provided on the plate or / and the pillar constituting the porous body. When bone begins to invade the cage porous body, osteoblasts grow from the truss structure body corresponding to the outermost surface of the cage, so the groove of the truss structure body can be expected to have the effect of promoting bone orientation from the initial stage of bone invasion. In order to fill the bone deeply in the thickness direction of the cage, the plate constituting the porous body can have a finite width so that the bone marrow fluid containing osteoblasts can be continuously supplied. With such a finite width, an appropriate gap can be provided between adjacent plates or / and pillars, and bone marrow fluid containing osteoblasts can be continuously supplied over the entire porous body. By this continuous supply, it is possible to realize bone invasion deep into the cage porous body. In addition, the porous body can be provided with holes on the side surface of the cage. These holes play a role in suppressing the retention of bone marrow fluid in the deep part of the porous body and promoting continuous circulation of bone marrow fluid.

In order to confirm the functionality of the above cage porous body, the porous body was implanted in the vertebrae of sheep, and the bone mass, bone orientation, and bone fixation in the porous body were evaluated by a pull out test. The results are shown below.

### [Evaluation sample]

In this evaluation, a sample was prepared in which three finite plates connected radially every 120 degrees as a porous body were arranged in a staggered pattern. The groove provided on the finite plate has a width of 0.2 mm and a depth of 0.15 mm. The plate thickness was 0.5 mm, and the grooves were arranged on both sides of the plate so that the bottoms of the grooves did not overlap each other. The outer shape of the cage was cylindrical for vertebra implantation. A triangular shape was adopted as the truss structure. The triangular truss structure communicated with the finite plates arranged in a radial pattern, and two kinds of the inscribed circles were set to 500 µm (Evaluation sample A) or 1000 µm (evaluation sample B). In order to investigate the influence of the external environment such as the conventional bone graft method and mechanical stimulation, the following evaluation samples C to E with holes on the side surface of the cage were prepared (Fig. 4). FIG. 4 (a) shows a conventional implant material (Evaluation sample C). FIG. 4 (b) shows an implant material according to an embodiment of the present invention in which a unidirectional hole penetrates (Evaluation sample D). FIG. 4 (c) shows a view of the implant material of FIG. 4 (b) as viewed from an angle rotated by 90 degrees, and shows holes on the side surface of the cage. (Evaluation sample E). In FIG. 4, it is shown that 10 is a transplanted bone, 11 is a hole on the side of the cage, 12 is a groove, 13 is a plate, and 14 is a pillar, respectively.

Sample C: Similar to a conventional cage, the cage is provided with only a through hole, and the hole is filled with a bone implant.
Sample D: In the cage having the porous body, the sheep head tail axis corresponding to the load transmission direction and the groove of the porous body are embedded in parallel.
Sample E: A cage having the porous body, which is embedded so that the head tail axis of the sheep and the groove of the porous body are perpendicular to each other. The cage shape is the same as sample D, but the load transfer direction is 90 degrees different from the groove of the porous body.

### [Sample preparation]

The above evaluation sample was designed with CAD software, output in STL format (Stereolithography), and integrally modeled by AM (Additive Manufacturing) using the data. The material used was Ti-6Al-4V alloy, which is a titanium-based alloy that has a proven track record as an implant material, and was modeled with a laser metal molding machine (EOS M290, manufactured by EOS). The groove width and thickness after modeling were 0.1 to 0.5 mm in width and 0.1 to 0.2 mm in depth with respect to the design values (width 0.2 mm, depth 0.15 mm).

### [Sheep burial]

Sample one by one was implanted in each of L1 to L4 of the lumbar vertebrae of Suffolk sheep 12 months or older. L1 to L4 vertebrae in which the cage was implanted were collected.

### [Observation of bone tissue]

In order to evaluate the bone induced inside the porous body of the cage, Vilanueva staining was performed, and a non-decalcified thin sections were prepared with a cross section parallel to the cranio-caudal axis with respect to the central part. The ratio of bone (BV: Bone Volume) to the space (TV: Total Volume) inside the cage was measured. The results are shown in Table 1.

**[Table 1]**

| Evaluation sample | Inscribed circle (µm) | Side surface hole | Buried direction | BV/TV (%) |
|---|---|---|---|---|
| A | 500 | None | Parallel to the cranio-caudal axis | 8.6 |
| B | 1000 | None | Parallel to the cranio-caudal axis | 13.3 |
| C | - | Existence | Parallel to the cranio-caudal axis | 8.2 |
| D | 1000 | Existence | Parallel to the cranio-caudal axis | 28.3 |
| E | 1000 | Existence | Vertical to the cranio-caudal axis | 25.9 |

From Table 1, in the inscribed circles of 500 µm and 1000 µm (evaluation samples A and B), 1000 µm has more bone mass, and the larger inscribed circle is more advantageous for bone invasion. In addition, in the comparison with / without holes on the side surface of the cage with an inscribed circle of 1000 µm (evaluation samples A and D), the bone mass was significantly larger with holes on the side surface of the cage. No significant difference was observed in the influence of the direction of the porous body (evaluation samples D and E).

### [Pull-out test]

A pull-out test was carried out with the evaluation sample C simulating a cage for conventional bone grafting and the evaluation samples D and E with the porous body, and the adhesion strength between the bone and the cage by the porous body was evaluated. After thawing the vertebra in which the cage was implanted, the vertebra was fixed with bone cement so that the screws provided in the cage part at the top of the jig are exposed. For the jig, a pull-out test of the cage was carried out with a tensile tester (manufactured by INSTRON, model number 5965). The crosshead speed was 5 mm / min, and the maximum load measured during the test was taken as the pull-out strength.

The results are shown in Fig. 5. FIG. 5 shows the result of the pull-out test with the maximum load measured at the time of the pull-out test as the pull-out strength. From FIG. 5, the pull-out strength of the evaluation samples D and E having the porous body is significantly higher than that of the conventional evaluation sample C requiring bone grafting. At 8 and 16 weeks, the pull-out strength of the evaluation sample C increased, but it was not as good as that of the evaluation samples D and E. It is presumed that this is a result of the porous body introducing bone having good bone quality into the porous body at an early stage. In addition, in the evaluation sample D implanted parallel to the cranio-caudal axis, the pull-out strength tends to increase as the age of the week increases. It is presumed that this is because the bone orientation is further promoted by the mechanical stimulation by the load in the principal stress direction of the mechanical environment in the body (in the sheep, the same cranio-caudal axis direction as in humans).

### [Orientation measurement]

In order to evaluate bone orientation, sliced sections of evaluation sample E prepared by bone tissue observation were used, and the orientation of collagen fibers related to bone orientation was analyzed by birefringence technique (WPA-micro: Photonic Lattice). FIG. 6 shows the observation results of the evaluation sample E. FIG. 6 (b) shows an enlarged view of a portion surrounded by a rectangle on the left side of the center of FIG. 6 (a) . As a result, it can be seen that the surrounding bones (sheep vertebrae) of the evaluation sample are oriented horizontally with respect to the figure, but the inside of the porous body is oriented vertically from the entrance. This indicates that the porous body itself induces an orientation different from the orientation inherent in the vertebrae of the sheep.

Further, FIG. 7 shows an example of using the implant material in one embodiment of the present invention. In FIG. 7, it is shown that 15 is a hole, 30 is a thickness direction, 31 is a cage in another aspect of the present invention, and 32 is a cage insertion direction, respectively. Although not shown, the hole 15 is also provided with a groove in this embodiment. The implant material is an example of a human cage, but the thickness direction is the human head-to-tail axis direction as shown in the figure. That is, the thickness direction can be the gap direction between the vertebrae. Since the evaluation sample this time has a cylindrical shape, In the evaluation sample D, the direction is the communication hole direction (head-to-tail axis direction) shown in the figure.

FIG. 8 shows a cross section parallel to the long axis of the cylinder when a cylindrical cage is used in the implant material according to the embodiment of the present invention. FIG. 8 (a) shows the central portion of the cylindrical cross section, FIG. 8 (b) shows the intermediate portion located between the central portion and the upper portion of the cylindrical cross section, and FIG. 8 (c) shows the upper portion of the cylindrical cross section, respectively. These implant materials can also be an embodiment of the present invention. That is, FIG. 8 shows a cross section of the upper part / middle part / central part parallel to the long axis of the cylinder. The internal structure is lined with tetra-shaped plates having grooves (central cross section), and these tetras can be connected by a truss structure (upper cross section) at the upper and lower parts of the cage.

As described above, according to the present invention, it can be seen that the geometric pattern structure itself of the internal structure having a groove (such as the above-mentioned tetra structure) can promote the formation of oriented new bone into the porous body regardless of the external environment such as mechanical stimulation.

From the above results, the relationship between the groove provided in the porous body and the bone orientation is clear. In particular, high pull-out strength was obtained in the evaluation sample E arranged so that the groove of the porous body was perpendicular to the principal stress direction in the biomechanical environment. This indicates that the geometric pattern of the porous body itself promotes bone orientation, independent of the biomechanical environment. Further, from the above, it was found that the groove width of 0.25 to 500 µm can be preferably used in consideration of the detectability of the osteoblast orientation direction and the size of the cell itself, the inscribed circle of the porous body can be 500 µm or more, and the plate thickness of 0.5 to 1 mm can be preferably used in consideration of the strength of the porous body and the groove depth.

### Example 2

Next, a box-shaped cage was designed as the implant material of the present invention, and the effect of inducing bone orientation was investigated. Specifically, as a large animal test (sheep), an extrusion test of a box-type cage placed between the sheep's vertebrae was performed. That is, in a large animal test using sheep, a box-type cage was implanted in the intervertebral space as in human clinical practice, and an extrusion test of the box-type cage was performed 8 weeks after implantation. For two types of box-shaped cage having heights of 8 mm and 11 mm, a comparative extrusion test was performed between the transplanted bone group of the box-shaped cage equivalent to the evaluation sample C of FIG. 4 (a) (the transplanted bone was pre-filled in the cage) and the bone orientation induction group equivalent to the evaluation sample D of FIG. 4 (b).

FIG. 9 shows a schematic view of the implant material according to one embodiment of the present invention when a box-shaped cage is used for the vertebral body. FIG. 9 (a) is a view seen from the lateral direction (direction perpendicular to the cranio-caudal direction) when an example of the implant material of the present invention is incorporated into the spine. FIG. 9 (b) is a schematic view of an example of the implant material of the present invention as viewed from the lateral direction (direction perpendicular to the cranio-caudal direction). In FIG. 9 (b), it is shown that 41 is the ceiling structure of the box-shaped cage (showing the state of bending) forming the contact surface with the vertebral body and the end plate in the vertical direction, and 42 is the pillar structure forming the oriented porous body, 43 is a vertebral body, 44 is a state in which the contact surface of the box-shaped cage (implant material in the example of the present invention) fits into the shape of the vertebral body end plate, 45 is a box-shaped cage and 46 are the end plates, respectively. FIG. 9 (c) is a cross-sectional view of an example of the implant material of the present invention as viewed from the lateral direction (direction perpendicular to the cranio-caudal direction). FIG. 9 (d) is a perspective view (viewed from diagonally above) of an example of the implant material of the present invention. It can be seen that the contact surface of the box-shaped cage fits into the shape of the vertebral end plate, and the contact of the oriented porous materials of the box-shaped cage further promotes bone fusion. It was also found that the wider the contact surface, the more the bone fusion is promoted.

FIG. 10 shows a schematic view of a box-shaped cage in the implant material according to one embodiment of the present invention. FIG. 10 (a) is a cross-sectional view of an example of the implant material of the present invention. FIG. 10 (b) is a schematic view of an example of the implant material of the present invention as viewed from the lateral direction (direction perpendicular to the cranio-caudal direction). In FIG. 10, it is shown that 51 is the ceiling structure of the box-shaped cage that forms the contact surface with the vertebral body and the end plate in the vertical direction, and 52 is the pillar structure that forms the oriented porous material, respectively. FIG. 10 (c) is a perspective view of an example of the implant material of the present invention. The ceiling structure of the box-shaped cage, which forms a contact surface with the vertebral body and the end plate in the vertical direction, may be in any shape such as a plate shape, a board shape, or a columnar shape as long as it is a flexible structure.

FIG. 11 is a diagram showing the results of comparative extrusion tests of the transplanted bone group and the bone orientation induction group for two types of box-shaped cages having heights of 8 mm and 11 mm. FIG. 11 (a) shows the result when both the box-shaped cage having heights of 8 mm and 11 mm are combined, FIG. 11 (b) shows the result when the height of the box-shaped cage is 8 mm, and FIG. 11 (c) shows the results when the height of the box-shaped cage is 11 mm, respectively.

From these results, the Paired T-test was performed for the three cases of height 8 mm, height 11 mm, and both cases, and significant differences were observed in all cases. It was found that the extrusion load of the bone orientation-induced porous body was higher than that of the transplanted bone, and the shear strength at the joint surface between the bone and the bone orientation derivative was significantly higher than that of the transplanted bone. That is, it was found that the extrusion strength of the box-shaped cage having the oriented porous material was significantly higher than that of the transplanted bone.

In Example 2, a box-shaped cage can be implanted in the vertebrae as a verification experiment, and when used in humans, the damaged intervertebral disc (between the vertebrae) can be removed, and the box-shaped cage can be implanted and fixed between the vertebrae. The direction of deflection is the cranio-caudal direction (the vertical direction in which the weight head is applied when the human is standing), and the design can be based on the assumption that the tetra-shaped structure body is compressed by the vertebrae above and below the cage and bends by compression. In the box-shaped cage, the structure body connecting the tetra-shaped structures body is thinly designed (0.5 mm), and the structure body itself bends of flexs so that the contact surface with the vertebrae, that is, the effect that the front and back surfaces of the box-shaped cage fit into the shape of the bone can be expected. By adapting to the shape of the bone, the front and back surfaces of the cage are in close contact with the vertebrae, making it easier to guide the bone into the porous body.

### Industrial applicability

According to the present invention, it can be expected to contribute to the fields of treatment of hard tissue diseases, regenerative medicine and dentistry (particularly orthopedics, neurosurgery, dentistry) and basic medicine.

### Description of the reference numerals

1 a plate thickness
2 an inscribed circle between plates
3 a groove width
4 a groove depth
5 the surrounding bone
6 the hole (second hole) communicating the surrounding bone and the inside of the implant material
7 the inside of the implant material (inside the porous body)
8 the hole communicating between the holes inside the implant material (third hole)
10 a transplanted bone
11 a hole on the side of the cage
12 a groove
13 a plate
14 a pillar
15 a hole (first hole)
30 a thickness direction
31 a cage in another aspect of the present invention
32 a cage insertion direction
41, 51 the ceiling structure of the box-shaped cage forming the contact surface with the vertebral body and the end plate
42, 52 the pillar structure forming the oriented porous body
43 a vertebral body
44 a state in which the contact surface of the box-shaped cage (implant material in the example of the present invention) fits into the shape of the vertebral body end plate
45 a box-shaped cage
46 the end plates

## Claims

1. An implant material **characterized by** comprising a hole (2) in at least one direction, and a member constituting the hole comprising grooves (12) wherein the grooves are provided on the surface of the hole in the depth direction of the hole.

2. An implant material according to claim 1, wherein the member constituting the hole is composed of a pillar (14) and/or a plate (13).

3. An implant material according to claim 1 or 2, wherein the grooves are provided in the pillars and/or the plates (13)

4. An implant material according to any one of claims 1 to 3, wherein the multiple holes are composed of pillars and / or plates (13) .

5. An implant material according to any one of claims 1 to 4, wherein the grooves are alternately arranged on the front and back surfaces of the pillar and / or the plate.

6. An implant material according to claim 2, wherein the pillar and / or the plate are composed of a simple unit and / or a block.

7. An implant material according to claim 2, wherein the pillar and / or plate are designed to bend.

8. An implant material according to any one of claims 1 to 7, wherein the hole is designed not to penetrate the implant material or is designed to penetrate the implant material.

9. An implant material according to any one of claims 1 to 8, wherein the implant material is at least one selected from a polymer material, a ceramic material, a metal material, an amorphous material, or a mixed material thereof.

10. An implant material according to any one of claims 1 to 9, wherein a width (3) of the groove is 0.25 to 500 µm.

11. An implant material according to any one of claims 1 to 10, wherein the holes are in communication with each other when there are a plurality of holes.

12. An implant material according to claim 7, wherein pillars and / or plates are designed so that the pillars and / or plates are bend by having a stretchable structure by themselves, or by varying the thickness, width, or height of the pillars or plates.

13. An implant material according to any one of claims 1 to 12, wherein the implant material is designed to bend at least a part of the contact surface where the implant material and a living body come into contact with each other.

14. An implant material according to any one of claims 1 to 13, wherein the hole is formed of a truss structure body and the inscribed circle (2) of the hole is 500 µm to 2000 µm.

15. An implant material according to any one of claims 1 to 14, wherein the implant material has a cage-like structure and has a second hole on a side surface of the cage-like structure.

16. A method of manufacturing the implant material according to any one of claims 1 to 15 **characterized by** being produced by a 3D modeling method.

## Patentansprüche

1. Implantatmaterial, **dadurch gekennzeichnet, dass** es ein Loch (2) in mindestens einer Richtung und ein das Loch bildendes Element mit Nuten (12) umfasst, wobei die Nuten auf der Oberfläche des Lochs in der Tiefenrichtung des Lochs vorgesehen sind.

2. Implantatmaterial nach Anspruch 1, wobei das Element, das das Loch bildet, aus einem Pfeiler (14) und/oder einer Platte (13) besteht.

3. Implantatmaterial nach Anspruch 1 oder 2, wobei die Nuten in den Pfeilern und/oder den Platten (13) vorgesehen sind.

4. Implantatmaterial nach einem der Ansprüche 1 bis 3, wobei die Mehrfachlöcher aus Pfeilern und/oder Platten (13) bestehen.

5. Implantatmaterial nach einem der Ansprüche 1 bis 4, wobei die Nuten abwechselnd auf den vorder- und rückwärtigen Oberflächen des Pfeilers und/oder der Platte angeordnet sind.

6. Implantatmaterial nach Anspruch 2, wobei der Pfeiler und/oder die Platte aus einer einfachen Einheit und/oder einem Block bestehen.

7. Implantatmaterial nach Anspruch 2, wobei der Pfeiler und/oder die Platte so gestaltet sind, dass sie sich biegen.

8. Implantatmaterial nach einem der Ansprüche 1 bis 7, wobei das Loch so gestaltet ist, dass es das Implantatmaterial nicht durchdringt oder so gestaltet ist, dass es das Implantatmaterial durchdringt.

9. Implantatmaterial nach einem der Ansprüche 1 bis 8, wobei das Implantatmaterial mindestens eines ist, das aus einem Polymermaterial, einem Keramikmaterial, einem Metallmaterial, einem amorphen Material oder einem gemischten Material davon ausgewählt ist.

10. Implantatmaterial nach einem der Ansprüche 1 bis 9, wobei die Breite (3) der Nut 0,25 bis 500 µm beträgt.

11. Implantatmaterial nach einem der Ansprüche 1 bis 10, wobei die Löcher miteinander in Kommunikation stehen, wenn eine Vielzahl von Löchern vorhanden ist.

12. Implantatmaterial nach Anspruch 7, wobei die Pfeiler und/oder Platten so gestaltet sind, dass die Pfeiler und/oder Platten durch eine dehnbare Struktur selbst oder durch Variation der Dicke, Breite oder Höhe der Pfeiler oder Platten gebogen werden.

13. Implantatmaterial nach einem der Ansprüche 1 bis 12, wobei das Implantatmaterial so gestaltet ist, dass es zumindest einen Teil der Kontaktoberfläche, an der das Implantatmaterial und ein lebender Körper miteinander in Kontakt kommen, biegt.

14. Implantatmaterial nach einem der Ansprüche 1 bis 13, wobei das Loch aus einem Gitterstrukturkörper gebildet ist und der eingeschriebene Kreis (2) des Lochs 500 µm bis 2000 µm beträgt.

15. Implantatmaterial nach einem der Ansprüche 1 bis 14, wobei das Implantatmaterial eine käfigartige Struktur und ein zweites Loch auf einer Seitenoberfläche der käfigartigen Struktur aufweist.

16. Verfahren zur Herstellung des Implantatmaterials nach einem der Ansprüche 1 bis 15, **dadurch gekennzeichnet, dass** es durch ein 3D-Modellierungsverfahren hergestellt wird.

## Revendications

1. Matériau d'implant **caractérisé en ce qu'**il comprend un trou (2) dans au moins une direction, et un élément constituant le trou comprenant des rainures (12), les rainures étant prévues sur la surface du trou dans le sens de la profondeur du trou.

2. Matériau d'implant selon la revendication 1, l'élément constituant le trou étant composé d'un pilier (14) et/ou d'une plaque (13).

3. Matériau d'implant selon la revendication 1 ou 2, les rainures étant prévues dans les piliers et/ou les plaques (13).

4. Matériau d'implant selon l'une quelconque des revendications 1 à 3, les trous multiples étant composés de piliers et/ou de plaques (13).

5. Matériau d'implant selon l'une quelconque des revendications 1 à 4, les rainures étant disposées alternativement sur les surfaces avant et arrière du pilier et/ou de la plaque.

6. Matériau d'implant selon la revendication 2, le pilier et/ou la plaque étant composés d'une unité simple et/ou d'un bloc.

7. Matériau d'implant selon la revendication 2, le pilier et/ou la plaque étant conçus pour se plier.

8. Matériau d'implant selon l'une quelconque des revendications 1 à 7, le trou étant conçu pour ne pas pénétrer dans le matériau d'implant ou pour le pénétrer.

9. Matériau d'implant selon l'une quelconque des revendications 1 à 8, le matériau d'implant étant au moins choisi parmi un matériau polymère, un matériau céramique, un matériau métallique, un matériau amorphe ou un mélange de ces matériaux.

10. Matériau d'implant selon l'une quelconque des revendications 1 à 9, une largeur (3) de la rainure étant comprise entre 0,25 et 500 µm.

11. Matériau d'implant selon l'une quelconque des revendications 1 à 10, les trous étant en communication les uns avec les autres lorsqu'il y a une pluralité de trous.

12. Matériau d'implant selon la revendication 7, les piliers et/ou les plaques étant conçus de manière à ce que les piliers et/ou les plaques se plient en ayant une structure extensible par eux-mêmes, ou en faisant varier l'épaisseur, la largeur ou la hauteur des piliers ou des plaques.

13. Matériau d'implant selon l'une quelconque des revendications 1 à 12, le matériau d'implant étant conçu pour plier au moins une partie de la surface de contact où le matériau d'implant et un corps vivant entrent en contact l'un avec l'autre.

14. Matériau d'implant selon l'une quelconque des revendications 1 à 13, le trou étant formé d'un corps de structure en treillis et le cercle inscrit (2) du trou est compris entre 500 et 2000 µm.

15. Matériau d'implant selon l'une quelconque des revendications 1 à 14, le matériau d'implant ayant une structure en forme de cage et comportant un second trou sur une surface latérale de la structure en forme de cage.

16. Procédé de fabrication du matériau d'implant selon l'une quelconque des revendications 1 à 15, **caractérisé par le fait qu'**il est produit par une méthode de modélisation 3D.
